# EUROPEAN PATENT SPECIFICATION

(11) **EP 2 992 887 B1**
(45) Date of publication and mention of the grant of the patent: **03.03.2021**
(21) Application number: 14788605.5
(22) Date of filing: 15.04.2014
(51) Int. Cl.: A61K 31/7004, A61Q 19/00, A23L 33/10, A23L 29/30, A23K 20/163, A61K 31/121, A61P 3/02, A61K 31/7012, A61K 31/7008, A61K 8/60, A23L 5/00, A23L 33/125, A61P 3/04, A61P 3/06, A61P 3/10, A61P 43/00

(54) **AGENT FOR ACCELERATING THE ENERGY EXPENDITURE AND/OR FOR IMPROVING THE DIMINISHED ENERGY EXPENDITURE FUNCTIONALITY**
MITTEL ZUR BESCHLEUNIGUN DES ENERGIEVERBRAUCHS UND/ODER ZUR VERBESSERUNG DER FUNKTIONSFÄHIGKEIT BEI VERMINDERTEM ENERGIEVERBRAUCH
AGENT POUR ACCÉLÉRER LA DÉPENSE ÉNERGÉTIQUE ET/OU POUR AMÉLIORER LA FONCTIONNALITÉ DE LA DÉPENSE ÉNERGÉTIQUE DIMINUÉE

(30) Priority: 26.04.2013 JP 2013093743
(43) Date of publication of application: 09.03.2016
(73) Proprietor: Matsutani Chemical Industry Co., Ltd., Itami-shi, Hyogo 664-8508 (JP)
(72) Inventor: YAMADA, Takako, Itami-shi Hyogo 664-8508 (JP); YOSHIKAWA, Yuko, Itami-shi Hyogo 664-8508 (JP); MATSUO, Tatsuhiro, Kita-gun Kagawa 761-0795 (JP); KIMURA, Tomonori, Itami-shi Hyogo 664-8508 (JP); HAYASHI, Noriko, Itami-shi Hyogo 664-8508 (JP)
(74) Representative: TBK
(86) International application number: PCT/JP2014/060715
(87) International publication number: WO 2014/175119

(56) References cited:
- WO-A1-2008/059625
- WO-A1-2013/035479
- JP-A- 2004 083 570
- JP-A- 2005 213 227
- JP-A- 2007 051 137
- JP-A- 2010 120 908
- CHUNG MIN-YU ET AL: "Hypoglycemic Health Benefits of D-Psicose", JOURNAL OF AGRICULTURAL AND FOOD CHEMISTRY, AMERICAN CHEMICAL SOCIETY, US, vol. 60, no. 4, 1 February 2012 (2012-02-01), pages 863-869, XP009192143, ISSN: 0021-8561, DOI: 10.1021/JF204050W [retrieved on 2011-12-22]
- IIDA T ET AL: "Failure of d-psicose absorbed in the small intestine to metabolize into energy and its low large intestinal fermentability in humans", METABOLISM, CLINICAL AND EXPERIMENTAL, W.B. SAUNDERS CO., PHILADELPHIA, PA, US, vol. 59, no. 2, 1 February 2010 (2010-02-01), pages 206-214, XP026850593, ISSN: 0026-0495 [retrieved on 2009-09-17]
- MATSUO T ET AL: "Less Body Fat Accumulation with D-Psicose Diet versus D-Fructose Diet", JOURNAL OF CLINICAL BIOCHEMISTRY AND NUTRITION, TOKYO, JP, vol. 30, 1 January 2001 (2001-01-01), pages 55-65, XP003003027, ISSN: 0912-0009
- MATSUO T ET AL: "D-psicose is a rare sugar that provides no energy to growing rats", JOURNAL OF NUTRITIONAL SCIENCE AND VITAMINOLOGY, UNIVERSITY OF TOKYO PRESS, TOKYO, JP, vol. 48, no. 1, 1 February 2002 (2002-02-01), pages 77-80, XP002973184, ISSN: 0301-4800
- OCHIAI M ET AL.: 'D-psicose increases energy expenditure and decreases body fat accumulation in rats fed a high-sucrose diet.' INTERNATIONAL JOURNAL OF FOOD SCIENCES AND NUTRITION vol. 65, no. 2, March 2014, pages 245 - 250, XP055289129
- OCHIAI M ET AL.: 'Anti-obesity effects of dietary d-psicose in growing rat fed a high- sucrose diet.' ANNALS OF NUTRITION AND METABOLISM vol. 63, no. SUPPL., September 2013, page 1575, XP008181372
- TETSUO IIDA ET AL.: 'Properties of Three rare Sugars D-psicose, D-allose, D- tagatose and Their Applications' OLEOSCIENCE vol. 13, no. 9, September 2013, pages 435 - 440, XP055289132

## Description

### Technical Field

The present invention relates to use of D-psicose for improving energy metabolism. Specifically, the present invention relates to use of D-psicose and/or derivatives thereof for promoting energy expenditure and/or preventing reduced energy consumption function.

### Background Art

Energy metabolism refers to input/output, conversion, and use of energy associated with biological activity, as exemplified by the process that decomposes and absorbs nutrients to produce adenosine triphosphate (ATP). Reduced or deteriorated energy metabolism is often seen in humans and other mammals, particularly in aged mammals, mammals at menopause, and mammals with health and other problems that cause diminished energy metabolism. Such mammals are characterized by poor heat production, and typically consume less energy than they take because of low basal metabolic rate. Because these animals fail to fully utilize the intake nutrients for heat production and convert it into combustion energy, the nutrients are partially converted into fat, and accumulate in fat cells.

Heat production tends to show an adaptive decrease, for example, during or after a diet therapy given to reduce body weight. This is the result of the body's response to deficient nutritional intake, automatically restricting the heat production activity, or switching to "saving mode." The reduced heat production typically remains at undesirably low levels even after the nutritional intake returns to sufficient levels, and the nutrients become restocked in the fat storage that has decreased during the diet therapy. The reduced energy expenditure in the body lowers the efficiency of medicaments or diet therapy given to reduce body weight. This increases the risk of many chronic diseases such as type II diabetes (insulin dysfunction), hyperlipidemia, arterial sclerosis, and high-blood pressure, with the result that the quality of life of the mammal suffers, and the lifespan of the animal decreases.

Concerning a method that has an effect on energy metabolism, PTL 1 proposes methods for one or more of enhancing energy metabolism, promoting healthy energy metabolism, maintaining healthy energy metabolism, preventing conditions that result in a decline or deficiency in energy metabolism, treating conditions that result in a decline or deficiency in energy metabolism, and preventing the accumulation of excess body fat in animals without reducing energy intake by the animals. The methods comprise administering isoflavones, not known to have an effect on energy metabolism, to the animals, preferably in amounts of from about 0.001 to about 10 g/kg/day.

PTL 2 discloses an energy expenditure accelerator containing chlorogenic acids or salts thereof as an active ingredient. PTL 3 discloses an energy expenditure accelerator containing a rice bran extract as an active ingredient, and proposes a method for safely accelerating energy expenditure.

PTL 4 discloses a nutritional composition containing a garcinia cambogia extract, a gymnema sylvestre leaf extract, and a green tea leaf extract, and proposes a method whereby consumption of the nutritional composition induces fast weight loss, burns calories, increases thermogenesis, supports energy metabolism, and/or suppresses appetite in individuals.

### Citation List

### Patent Literature

PTL 1: JP-T-2012-504554
PTL 2: JP-A-2010-241800
PTL 3: JP-A-2012-020941
PTL 4: JP-T-2007-535576
PTL 5: Japanese Patent No. 4609845
PTL 6: Japanese Patent No. 4724823
PTL 7: Japanese Patent No. 5171249
PTL 8: JP-A-06-125776
PTL 9: JP-A-2002-017392
PTL 10: WO2010/113785
PTL 11: JP-A-2007-91696

### Non Patent Literature

NPL 1: J. Am. Chem. Soc. 1955, 77, 3323-3325
NPL 2: Biochemistry, 16(10), 2169-75, 1977.

WO2008/059625 A1 describes a promoter for the migration of glucokinase from nucleus to cytoplasm which comprises D-psicose and/or D-tagatose as the active ingredient; or a composition for preventing or treating the onset of a medical condition relating to the glucokinase activity. This composition, which comprises D-psicose and/or D-tagatose as the active ingredient and is usable for preventing or treating the onset of a medical condition relating to the glucokinase activity, is in a form selected from the group consisting of a food additive, a food material, a food, a drink, a health food, a health drink, a drug and a feed. The medical condition relating to the glucokinase activity is selected from among impaired glucose tolerance, type 2 diabetes, insulin resistance disease, lipidemia, metabolic syndrome and obesity.

### Summary of Invention

### Technical Problem

Functions of D-Psicose are disclosed in various patents. PTL 5 discloses a physiological activator containing D-psicose as an active ingredient and selected from the group consisting of an antihyperglycemic agent, an anti-diabetic agent, a chemokine MCP-1 secretion inhibitor, an anti-arterial sclerosis agent, a microglial migration inhibitor, and an anti-ischemic cranial nerve disease agent. PTL 6 discloses an oxidative damage inhibitor for nigral dopaminergic neurons comprising D-psicose, or D-psicose and D-allose as active ingredients. PTL 7 discloses a composition for suppressing a circadian abnormal increase of blood plasma glucose concentration. The composition contains D-psicose and/or derivatives thereof as active ingredients, and suppresses an abrupt increase of glucose levels due to the constituent D-glucose of a digestive sugar ingested with food and drinks. The composition is given to mammals in need of suppression of abnormal blood plasma glucose concentration increase during a day so that the D-psicose and/or derivatives thereof are ingested regularly for at least 7 consecutive weeks to suppress abnormal blood plasma glucose concentration increase throughout a day.

As described above, there is some knowledge of various functions of D-psicose, including the antihyperglycemic effect, suppression of chemokine MCP-1 secretion, and suppression of microglial migration. However, D-psicose and derivatives thereof used to stimulate heat production as in the present invention have surprisingly different working profiles, and there is no publication that describes heat production accelerating effect. It is also unclear as to how much dose is effective.

There is also no solution that makes use of a food product that is effective for the detrimental decline of energy metabolism in humans and other mammals in need of the promotion of energy expenditure and/or the prevention of reduced energy consumption function.

The present invention is intended to provide a solution with the use of a functional food product that effectively enhances energy metabolism, specifically a rare sugar containing at least D-psicose.

There is also a need for a medicinal agent that can effectively treat and prevent diseases involving low basal metabolism and low body temperature.

Development of a health food and drink product or an animal feed that can treat and/or prevent diminished energy expenditure functionality through daily intake is also needed.

It is accordingly an object of the present invention to provide a solution to the foregoing problems, specifically to provide a food and drink product, a feed, a drug, a quasi drug, an oral composition, and a cosmetic for the promotion of energy expenditure and/or the prevention of reduced energy consumption function. The invention is also intended to provide a food and drink additive having the effect to promote energy expenditure and/or prevent reduced energy consumption function.

### Solution to Problem

The present inventors conducted intensive studies to solve the foregoing problems, and found a novel effect of rare sugar D-psicose to enhance energy metabolism. Specifically, it was found that D-psicose intake increases the breath energy expenditure at rest, and that D-psicose gives a warm sensation in the body when taken in at least 2.5 g/day for 2 consecutive weeks. The present invention was completed on the basis of these findings.

Specifically, the present invention includes the use of an agent as follows.

A first invention is an agent comprising D-psicose as an active ingredient for the use defined in claim 1.

In a second invention according to the first invention, the agent is added to a food and drink product, a feed, a drug, a quasi drug, an oral composition, a cosmetic, or a food and drink additive so that the D-psicose is taken in the form of a food and drink product, a feed, a drug, a quasi drug, an oral composition, a cosmetic, or a food and drink additive in 2.5 g or more for adults.

In a third invention according to the first or second invention, the agent is given to continuously administer the D-psicose at least once a day in 2.5 g or more for adults.

In a fourth invention according to any one of the first to third inventions, the D-psicose contained as an active ingredient results from alkali isomerization of glucose, fructose, and/or an isomerized sugar.

In a fifth invention according to any one of the first to fourth inventions, the D-psicose comprises D-psicose and/or derivatives thereof, the derivatives of D-psicose being selected from sugar alcohols in which the ketone group of D-psicose is converted into an alcohol group, uronic acids in which the alcohol group of D-psicose is oxidized, and amino sugars in which the alcohol group of D-psicose is substituted with an NH₂ group.

In a sixth invention according to any one of the first to fifth inventions, the agent is given to administer the D-psicose for at least 2 weeks with food in a daily dose of 2.5 g to 25 g for adults.

In a seventh invention according to any one of the first to sixth inventions, the agent is given to humans and other mammals in need of treatment and/or prevention of accelerated energy expenditure and/or diminished energy expenditure functionality.

### Advantageous Effects of Invention

The present invention can provide a solution with the use of a functional food product that effectively enhances energy metabolism, specifically a rare sugar containing at least D-psicose.

The agent for the use according to claim 1 is given in the form of a composition added to a food and drink product, a feed, a drug, a quasi drug, an oral composition, a cosmetic, or a food and drink additive (in the form of a food and drink product, a feed, a drug, a quasi drug, an oral composition, a cosmetic, or a food and drink additive). The composition is effective at preventing and/or improving low basal metabolism, and preventing and/or improving low body temperature. The nutritional composition of the present invention has high safety, and can be used for extended time periods even for aged individuals with weakened renal function, without putting an excessive protein load.

### Brief Description of Drawings

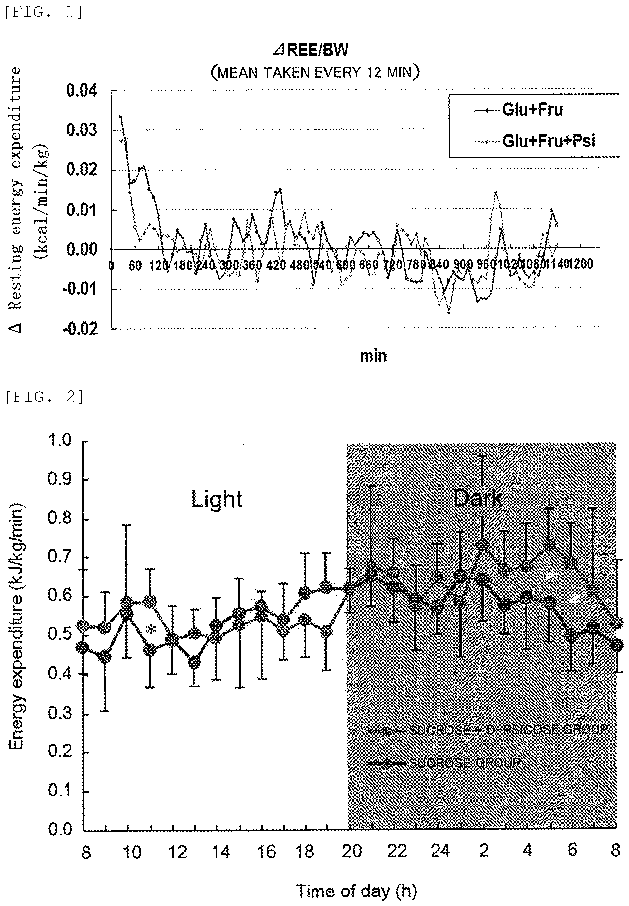

FIG. 1 represents the results of energy metabolism measurements in rats that had single oral administration of D-psicose according to Reference Example, in which each value represents energy metabolism per rat weight represented as a mean value taken every 12 minutes.
FIG. 2 shows the results of energy metabolism measurements by long-term administration of D-psicose in rats according to Example, representing energy metabolism as an hourly energy expenditure value per rat weight (weight at the time of measurement) in a light period and a dark period after 5 to 7 weeks from administration.

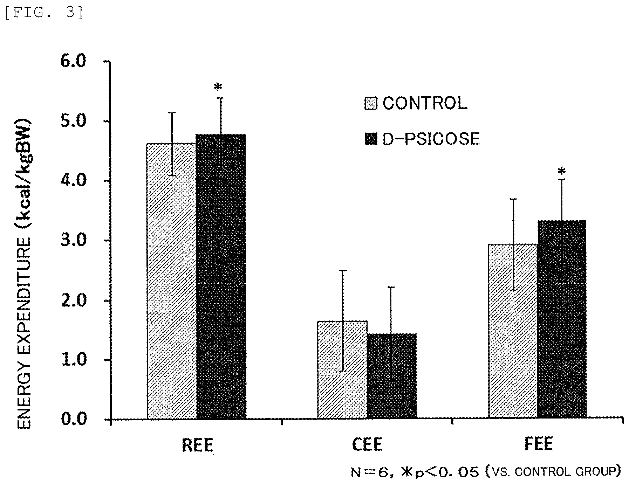

FIG. 3 shows the results of energy metabolism measurements by single D-psicose administration in humans according to Example, representing energy metabolism as resting energy expenditure (REE), carbohydrate combustion (CEE), and fat combustion (FEE) per body weight during a time period after 20 minutes to 4 hours from breakfast.

### Description of Embodiments

As used herein, "energy expenditure" is intended to include basal metabolism that expends energy to sustain life, such as breathing and regulation of body temperature, activity metabolism that expends energy trough exercise and work, and food-induced heat metabolism that expends energy through induction by ingestion of food.

The unexpended energy from the intake energy source accumulates as body fat. Some fat accumulation is necessary as an energy source, but excess accumulation increases the likelihood of various diseases, and should be avoided. It is accordingly desirable to have high energy expenditure in various forms of metabolism, including basal metabolism.

As used herein, "D-psicose" is a type of rare sugar, and may be enzymatically or chemically produced through epimerization from D-fructose (fructose), or indirectly from D-glucose (glucose), or may be extracted from plants. The extracted D-psicose may be purified completely, or may be partially purified to contain small amounts of impurities that become included during the production.

For example, it is relatively easy to prepare D-psicose by using an enzymatic technique with epimerase, as described in PTL 8. The chemical technique described in NPL 1 also may be used.

The D-psicose solution obtained by using these and other techniques may be optionally purified by using methods, for example, such as protein removal, decolorization, and desalting, and concentrated to collect D-psicose in the form of a syrup-like product. The product may be fractionated and purified by column chromatography to obtain a high-purity powder of 99% or higher purity. Such D-psicose products may be directly used in the form of monosaccharide.

As used herein, "rare sugars" are particular types of monosaccharides, the basic units of carbohydrates. Specifically, "rare sugars" as used herein are monosaccharides (aldoses, ketoses) that are found in much smaller quantities in nature than more abundant "natural monosaccharides" such as D-glucose. The "rare sugars" also include derivatives of such monosaccharides (sugar alcohols).

D-Glucose, D-galactose, D-mannose, D-ribose, D-xylose, and L-arabinose are six of the aldoses that are typically abundant in nature, whereas other aldoses are much less abundant, and are defined as rare sugars. D-Fructose is a ketose that is relatively abundant in nature, and other ketoses can classify as rare sugars. Examples of such rare ketoses include D-psicose, D-tagatose, D-sorbose, L-fructose, L-psicose, L-tagatose, and L-sorbose. Sugar alcohols can be produced through reduction of monosaccharides. D-Sorbitol is relatively abundant in nature, whereas other sugar alcohols are less abundant, and can classify as rare sugars.

At present, D-psicose and D-allose are two rare sugars that can be mass produced. D-Psicose is a ketohexose, and D-allose is an aldohexose. D-Psicose can be obtained by using any means, including extraction from nature, and chemical or biological synthesis. D-Allose can be enzymatically obtained from D-psicose in a D-psicose-containing solution acted upon by D-xylose isomerase (PTL 9).

Instead of being used in powder form such as above, the D-psicose of the present invention may be used in the form of an isomerized sugar-containing syrup. Such syrup is readily available as the commercially available product "Rare Sugar Sweet" (distributed by RareSweet; sold by Matsutani Chemical Industry Co., Ltd.).

Rare Sugar Sweet is a syrup produced from a raw material isomerized sugar, and contains rare sugars obtained by using the technique disclosed in PTL 10. The rare sugars contained in this product are primarily D-psicose and D-allose. The rare sugars contained in the rare sugar-containing syrup obtained by using the technique of the foregoing publication are 0.5 to 17 mass% D-psicose, and 0.2 to 10 mass% D-allose with respect to all sugars. The syrup also contains unidentified sugars.

The method for obtaining the rare sugar-containing syrup is not limited to the foregoing technique. The term "rare sugar-containing syrup" encompasses a wide range of syrups containing various monosaccharides (including rare sugars) obtained by reacting monosaccharides (D-glucose or D-fructose) under alkali conditions in the Lobry de Bruyn-van Ekenstein transformation reaction or the retro aldol reaction first reported in late 19th century, followed by an aldol reaction (these reactions are referred to as alkali isomerization reactions). Typically, the raw material D-glucose and/or D-fructose are alkali isomerized until the D-glucose and/or D-fructose contents in the syrup become 55 to 99 mass%.

Of various methods available for rare sugar measurements, high-performance liquid chromatography is most commonly used for the separation and measurement of rare sugars. As an example, measurement may be performed under the conditions described in PTL 9 (detector: RI; column: Mitsubishi Kasei MCI GEL CK 08EC; column temperature: 80°C; mobile phase: purified water; mobile phase flow rate: 0.4 mL/min; sample injection amount: 10 µL).

Examples of raw materials used for the production of the rare sugar-containing syrup include starch, sucrose, isomerized sugar, fructose, and glucose. Isomerized sugar can be widely regarded as a mixed sugar that contains D-glucose and D-fructose as principal components in a specific composition ratio. Typically, isomerized sugar refers to liquid sugars containing glucose and fructose as main components, prepared by glucose isomerase- or alkali-catalyzed isomerization of a sugar solution of primarily glucose obtained by enzyme (e.g., amylase) or acid hydrolysis of starch. The JAS standards terms isomerized sugars as "glucose fructose liquid sugar" for sugars with the fructose content (the proportion of fructose in the sugar) of less than 50%, "fructose glucose liquid sugar" for sugars with the fructose content of 50% or more and less than 90%, "high fructose liquid sugar" for sugars with the fructose content of 90% or more, and "sucrose-mixed fructose glucose liquid sugar" for sugars obtained by adding sucrose to the glucose fructose liquid sugar in amounts that do not exceed the glucose fructose liquid sugar. The raw materials of the rare sugar-containing syrup used in the present invention may be any of such isomerized sugars.

For example, a rare sugar-containing syrup prepared from D-fructose contains 5.2% D-psicose, 1.8% D-allose, 15.0% glucose, and 69.3% D-fructose. A rare sugar-containing syrup prepared from isomerized sugar contains 3.7% D-psicose, 1.5% D-allose, 45.9% glucose, and 37.7% D-fructose. When the raw material is D-glucose, the product syrup contains 5.7% D-psicose, 2.7% D-allose, 47.4% glucose, and 32.1% D-fructose. However, the sugar composition depends on the raw material and the processing method used.

The D-psicose used in the present invention may be used as D-psicose and/or derivatives thereof.

Derivatives are a collective term for compounds resulting from transformation of the molecular structure of a starting compound through chemical reaction. Taking hexose as an example, the derivatives include sugar alcohols and amino sugars. In the case of D-psicose, the derivatives include sugar alcohols in which the ketone group has been converted into an alcohol group, uronic acids in which the alcohol group has been oxidized, and amino sugars in which the alcohol group has been substituted with an NH₂ group.

Enzyme specificity represents the primary mechanism of action in the physiological effects of monosaccharides, and sugar conformation is particularly important. As an example, the enzyme fructokinase in liver catalyzes phosphorylation by acting mainly on the substrate fructose. This enzyme also phosphorylates D-tagatose and D-psicose, C-3 isomers of fructose. However, the phosphorylation rate is known to vary from conformation to conformation (NPL 2). The reaction rate of the enzyme is affected particularly by whether the carbon atoms at positions 2, 3, 4, and 5 are trans or axial. It thus appears likely that the effects of the present invention can be sufficiently obtained even with derivatives, provided that the conformation is maintained to some extent.

The D-psicose used in the present invention may be used as a mixed sugar, for example, a mixed sugar of D-glucose, D-fructose, and other rare sugars (e.g., allose).

It was found, rather surprisingly, that D-psicose and derivatives thereof, when taken continuously, have the effect to accelerate energy expenditure in relatively large mammals, including humans.

D-Psicose and derivatives thereof are continuously taken at least once a day in 2.5 g or more for adults in terms of a D-psicose amount. The daily dose is 2.5 g to 25 g, preferably 2.5 g to 20 g for adults, and D-psicose and derivatives thereof may be given once a day, or 2 or 3 times a day with appropriate intervals, before, after, or during meals. A dose below 2.5 g is ineffective, whereas a dose in excess of 25 g is also undesirable as it adds to the daily cost. The dose may be decided taking into consideration factors such as which biometabolism parameter needs to be reduced to what extent, and whether a body weight is within a standard body weight range.

By giving D-psicose and derivatives thereof before, after, or during meals, the energy expenditure can increase more than when D-psicose and derivatives thereof are not given. The energy expenditure accelerating effect becomes more desirable when D-psicose and derivatives thereof are given continuously. The preferred continuous dosing period is 2 weeks, or longer than 2 weeks.

The D-psicose and derivatives thereof used in the present invention may have any form, including solids (such as a powder, a fine powder, a granule, a crystal, and a tablet), aqueous solutions, and solutions. The producing process is not particularly limited either. It is also possible contain other sweetness components, or non-taste components such as a bulking agent and a carrier, provided that these do not interfere with the object of the present invention.

The present invention also includes food and drink products, feeds, drugs, quasi drugs, oral compositions, cosmetics, and food and drink additives prepared with materials, particularly functional materials, containing the D-psicose-containing composition. Specifically, the D-psicose and derivatives thereof is used by being added to a food and drink product, a feed, a drug, a quasi drug, an oral composition, a cosmetic, or a food and drink additive so that the D-psicose is taken in the form of a food and drink product, a feed, a drug, an oral composition, or a food and drink additive in 2.5 g or more per administration per unit preparation for adults.

Examples of food and drink products used in the present invention include soy sauce, soy sauce powder, *miso, miso* powder, *moromi, hishio, furikake,* mayonnaise, dressing, vinegar, *sanbaizu* (vinegar mixture), sushi vinegar powder, taste enhancer for Chinese food, *tentsuyu* (dipping sauce for *tempura*), *mentsuyu* (dipping sauce for noodles), sauce, ketchup, sauce for grilled meat, curry block, stew powder, soup powder, soup stock powder, mixed seasoning, *mirin, shin-mirin,* table sugar, and coffee sugar. The present invention can be advantageously used as a sweetener, a taste enhancer, or a quality enhancer for these and other seasonings.

The present invention also can be used as an additive for various food and drink products, including, for example, Japanese confectioneries (such as *senbei, arare, okoshi,* rice cake, *manjyu, uirou,* red bean pastes, *yokan, mizuyokan, kingyoku,* jelly, castella, and candies), Western confectioneries (such as bread, biscuit, cracker, cookie, pie, pudding, butter cream, custard cream, profiterole, waffle, sponge cake, doughnut, chocolate, chewing gum, caramel, and candies), frozen desserts (such as ice cream, and sherbet), syrups (such as fruit syrup, and *kourimitsu* (sugar boiled down with the white of eggs)), pastes (such as flour paste, peanut paste, and fruit paste), processed fruit and vegetable products (such as jams, marmalade, syrups, and confections), bread, noodles, cooked rice, processed cereal products (such as artificial meat), pickled vegetables (such as *fukujinzuke, bettarazuke, senmaizuke,* and *rakkyozuke),* pickles powders (such as *takuanzuke* powder, and *hakusaizuke* powder) , livestock products (such as ham, and sausage), fish meat products (such as fish ham, fish sausage, *kamaboko, chikuwa,* and *tempura),* delicacies (such as sea urchin, squid *shiokara, sukonbu, sakisurume,* and dried pufferfish with *mirin*)*, tsukudani* products (such as seaweed, *sansai,* dried shredded squid, small fish, and shellfish), side dish products (such as *nimame,* potato salad, and *konbu* roll), dairy products, bottled and canned products made from fish meat, meat, fruits, and vegetables, alcoholic beverages (such as *gouseishu* (sake with additives), fruit wine, Western liquors, and liqueur), soft drinks (such as coffee, hot chocolate, juice, carbonated drink, lactic acid drink, and lactobacillus beverage), premix powders (such as pudding mix, and hot cake mix), and instant food and drink products (such as instant juice, instant coffee, instant red bean soup, and instant soup).

Examples of functional drinks include carbonated drinks (such as cola), sports drinks, fruit juice drinks, milk beverages, and tea drinks. The most ideal target would probably be carbonated drinks, which cause obesity when taken in large quantities.

The food and drinks may be functional foods, dietary supplements, or food with health claims. The form of the food and drinks is not particularly limited. For example, the food may be produced with proteins of high nutritional value with balanced amino acids (such as milk protein, soybean protein, and egg albumin), decomposition products thereof, egg white oligopeptides, soybean hydrolysates, and a mixture of simple amino acids, using an ordinary method. The food and drinks also may be used in the form of, for example, soft capsules or tablets.

The functional food products or dietary supplements may be used in the form of, for example, liquid food, defined formula diet, elemental diet, nutritious supplement drink, capsule formulation, and enteric nutritional supplements containing materials such as carbohydrate, fat, trace elements, vitamins, emulsifiers, and flavoring ingredients. When the food and drinks are, for example, sports drinks or nutrition drinks, the nutritional balance or flavor may be improved by adding nutritional additives or compositions such as amino acids, vitamins, and minerals, or other additives such as spice, flavoring ingredients, and dyes.

When the composition of the present invention is used for food products, the composition may be used as it is, or may be prepared in the form of a dilution in oil, or a milky liquid food, or may be used with a carrier commonly used in food industry. The drinks are in the form of non-alcoholic drinks or alcoholic drinks. Examples of non-alcoholic drinks include carbonated drinks, non-carbonated drinks (such as fruit juice, and nectar drinks), soft drinks, sports drinks, tea, coffee, and hot chocolate. Examples of alcoholic drinks include common alcoholic beverages such as medicated liquor, *chuhai, umeshu,* beer, low-malt beer, and alternative beer.

When used in the form of a food material or a food additive for the purpose of improving its biological effects, the composition of the present invention may be used in the form of a tablet or a capsule formulation, or a powder, a granule, or other such solid agents used by being dissolved in drinks; semi-solids such as a jelly; liquids such as drinking water; or a high-concentration solution that is diluted before use.

The composition of the present invention also may be appropriately added to food products to prepare supplement food or hospital food intended to improve biological functions. It is also possible to appropriately mix additional components, for example, such as vitamins, carbohydrates, dyes, and flavoring ingredients commonly added to food products. The food products may be eaten in any form, including liquid and solid forms. The composition of the present invention also may be taken in the form of a soft capsule formulation by being encapsulated with a coating such as gelatin. The capsules are prepared, for example, with a gelatin coating prepared by dissolving the raw material gelatin in water, and adding a plasticizer (such as glycerine, D-sorbitol) to the gelatin solution.

The composition of the present invention is also applicable to feeds for domestic animals, poultry, and pets. For example, the sweetener composition of the present invention may be mixed in dry dog food, dry cat food, wet dog food, wet cat food, semi-moist dog food, poultry feed, or feeds for domestic animals such as cows and pigs. The feed itself may be prepared by using an ordinary method.

The therapeutic agents and preventive agents may also be used for non-human animals, including, for example, domesticated mammals such as cows, horses, pigs, and sheep; birds such as chicken, Japanese quail, and ostrich; pets such as reptiles, birds, and small mammals; and farmed fish.

A medicinal agent that takes advantage of the effects of the composition of the present invention may be used alone, or may be typically mixed with suitable additives such as an excipient, a stabilizer, a preservative, a binder, and a disintegrant, and prepared into a suitably selected dosage form such as a liquid, a granule, a subtle granule, a powder, a tablet, a capsule formulation, a pill, an ointment, a patch, an aerosol spray, a spray, and an injection. Such medicinal agents or preparations may be administered orally, transnasaly, percutaneously, or transvenously.

The composition of the present invention may be prepared into a medicinal agent using an organic or an inorganic solid, semi-solid or liquid carrier for medical use, a solubilizer, or a diluent as may be suitably selected for oral administration, transnasal administration, transdermal administration, or transvenous administration. For example, the composition of the present invention may be directly sent into the intestines or stomach through a tube in patients having difficulty with oral administration, whereas the composition of the present invention may be given as a food and drink product when it can be taken orally.

The carrier used for medicinal agents containing the composition of the present invention may be any of water, gelatin, lactose, starch, magnesium stearate, talc, animal and vegetable oils, benzyl alcohol, gum, polyalkylene glycol, petroleum resin, coconut oil, lanolin, and all other carriers used for medicinal applications. A stabilizer, a humectant, an emulsifier, or salts for varying osmotic pressure or maintaining a compounded agent at an appropriate pH may be appropriately used as an auxiliary medicinal agent.

A soluble film is used for preparation of cosmetics and other products. For example, an edible soluble film, such as a flavor film with a flavoring ingredient, has been used for purposes such as refreshing and prevention of bad breath. There are also ideas to use, for example, a moisturized cosmetic film as a mask, or dissolve such a film in water for use as an emulsion. There are also studies to use a soluble film as a plaster retaining an anti-inflammatory agent. There is also proposed a soluble film of desirable solubility and desirable film characteristics preferred for use as a wrapping material for food products, drugs, and other applications, or a carrier for retaining food products, and active ingredients of drugs (PTL 11). The composition of the present invention also has application in drugs, quasi drugs, and cosmetics, as above.

Reduction of basal metabolism in the elderly is believed to be the result of a reduced muscle mass and the corresponding reduction of generated body heat due to, in large part, aging and the accompanying decline of protein synthesis. The reduced basal metabolism may cause loss of appetite, and the insufficient nutritional intake may further weaken protein synthesis in a vicious circle.

The composition of the present invention is useful for the prevention and/or improvement of such reduction of basal energy expenditure.

Individuals with hypothermia involve bad blood circulation, which may lead to weakened immunity, and cause various diseases, including lifestyle-related disease. The ability to decompose and synthesize protein also weakens, and the body fails to make the necessary building blocks.

The ability to regulate body temperature is also weak in the elderly, and these individuals have difficulty raising their body temperature. The composition of the present invention is also useful for the prevention and/or improvement of low basal body temperature.

Obesity is a condition involving excessive accumulation of fat tissue. A moderate amount of fat tissue is essential for functions such as energy supply, insulation to maintain body temperature, and protection of internal organs. However, excess fat accumulation increases the likelihood of various health problems. Particularly, excessive storage of visceral fat around internal organs needs to be carefully monitored as it may cause diseases such as diabetes, stroke, and myocardial infarction.

People with obesity often involve high blood pressure and/or heart problems, and a medicinal agent that enhances the heat production energy expenditure without increasing blood pressure is particularly advantageous for obese patients.

The composition of the present invention is useful for the prevention and/or improvement of obesity and visceral fat accumulation.

There are indications that excessive accumulation of visceral fat lowers the secretion of beneficial adipocytokine, or adiponectin as it is also called, from the visceral fat. Low adiponectin lowers the effects of insulin, and reduces the transport of glucose into cells from blood. This increases the blood glucose level (hyperglycemia). Hyperglycemia is known to involve various complications such as diabetes, kidney problems, nerve damage, and myocardial damage.

The nutritional composition according to the present invention is useful for the prevention and/or improvement of hyperglycemia involving increased visceral fat.

Increased visceral fat often accompanies abnormal lipid metabolism such as high LDL cholesteremia, hypertriglyceridemia, remnant hyperlipoproteinemia, high small dense LDL, and low HDL cholesteremia. These are conditions where the blood lipid level becomes abnormal, and the abnormal lipid metabolism increases the production of lipoperoxide lipids such as lipoperoxide LDL cholesterol, and increases the risk of atherosclerosis.

The composition of the present invention can effectively reduce the onset of abnormal lipid metabolism such as increased blood LDL cholesterol due to increased visceral fat, and is useful for the prevention and/or improvement of abnormal lipid metabolism associated with increased visceral fat.

As a rule, muscle mass tends to decrease with age. Intake of the composition of the present invention enhances the basal metabolism, and can stimulate appetite to increase protein intake, a source of muscles. The effect is particularly notable when the energy source of myoprotein synthesis is visceral fat, which is highly involved in abnormal lipid metabolism, hyperglycemia, fatty liver, and deteriorated liver function. The composition of the present invention thus greatly contributes to the elderly and bedridden individuals, for whom an effective health food and drink composition has not been available.

The composition of the present invention can preferably be used to increase the muscle mass of individuals with low muscle mass (for example, old or bedridden individuals), and/or to suppress reduction of muscle mass in these individuals.

The dose of the D-psicose and derivatives thereof varies from condition to condition, and, as is evident, may be adjusted according to the conditions to be treated, and the forms in which the D-psicose and derivatives thereof are used. For the treatment of inhibited and/or undesirably low heat production, D-psicose is given at least once a day in a target daily dose of typically 2.5 g to 30 g for adults, preferably in 30 g or less per day for adults, once a day or 2 or 3 times a day with appropriate intervals, before, after, or during meals. Desirably, D-psicose is formed into a preparation such that 2.5 g or more of D-psicose is contained per unit preparation. The effects do not develop when the dose is less than 2.5 g, whereas a dose in excess of 30 g is also not desirable as it tends to cause diarrhea.

### Examples

The present invention is described in detail below using Examples. The present invention is in no way limited by the following Examples.

### [Reference Example]

### Metabolism Test by Single Oral Administration of D-Psicose in Rats

### Experiment Methods

Effects of single oral administration of D-psicose on energy metabolism were examined using male Sprague-Dawley rats (7 weeks of age; purchased from CLEA Japan). The rats were preliminarily maintained for 1 week with a commercially available solid feed (CE-2; CLEA Japan), and were divided into 3 groups so as to be uniform in body weight upon measuring the body weight (each group contained eight rats). As shown in Table 1, the three groups are a glucose + fructose administered group (-Psi group), a glucose + fructose +psicose administered group (+Psi group), and a group that was given only water (water administered group).

The rats were individually put in a cage of a respiratory metabolism measurement system for small animals MK-5000 (Muromachi Kikai Co., Ltd.) at 16 o'clock on the day before a calorimetric test. The rats were measured by indirect calorimetry, and acclimated in the dark without food (dark period).

The rats were placed under a light at 10 o'clock next morning (light period) . The rats were each measured for body weight, acclimated for 3 hours, and forcibly administered with 5 ml/(300 g body weight) of a dosing material through mouth using a sonde (the dosing material for the +Psi group contained psicose, whereas the dosing material for the -Psi group did not contain psicose) . The dosing materials are shown in Table 1. The solution concentrations are different to match total energy amounts. Only water was given to the water administered group. For the rest of the experiment, the rats were allowed access to only purified water, and the calorific value was continuously measured until 10 o'clock next day at 23°C under light.

Resting energy metabolism (resting energy expenditure; hereinafter, "REE", kcal/min) was calculated by using the Weir equation, and the result was divided by body weight (hereinafter, "BW", in kg). The mean of measured values from 12 min to 60 min before the administration was regarded as a value at hour 0, and amounts of change were calculated over time. The values from each rat were averaged every 12 min. The values from the rats of the same group were averaged, and the mean value of data from the water administered group was subtracted from the data of the +Psi group (or the Psi group) in each time zone. The result is represented by AREE/BW.

To test significance, the +Psi group was tested for a significant difference against the -Psi group at each time point. The rats were first tested for equal variance in an F test, followed by a Student's t test when the data had equal variance, and an unequal variance t test (Welch's t test) when the data had unequal variance. The results were considered significant for p < 0.05 (two-tailed).

**[Table 1]**

| Group | Dosing material | Solution concentration (%) |
|---|---|---|
| -Psi group | Glucose^{*1} + fructose^{*2} | 25.0 |
| +Psi group | Glucose^{*1} + fructose^{*2} + psicose^{*3} | 26.8 |

| | | |
|---|---|---|
| *1 Glucose: High Grade D(+)-Glucose (Kishida Chemical product, Lot. B65704B) *2 Fructose: High Grade D(-)-Fructose (Kishida Chemical product, Lot. M17908U) *3 Psicose: D-Psicose (product from Izumorning Co., Ltd.) | | |

### Results

The results are shown in FIG. 1. There was no significant difference between the two groups for ΔREE/BW representing a mean value taken every 12 minutes. With food that was purely carbohydrate, the simultaneous D-psicose intake did not have effect on the energy expenditure of the rat immediately after the carbohydrate ingestion. The same results were obtained in human tests, in which single D-psicose administration with pure carbohydrate food did not have effect on energy expenditure.

### [Example 1]

### Metabolism Test by Long-Term Administration of D-Psicose in Rats

### Experiment Methods

Effects of long-term administration of D-psicose on energy metabolism were examined using male Wistar rats (3 weeks of age; purchased from Japan SLC). The rats were preliminarily maintained for 1 week with a commercially available powder feed (product name MF; Oriental Yeast Co., Ltd.), and were divided into 2 groups so as to be uniform in body weight upon measuring the body weight (each group contained eight rats). The two groups are a sucrose administered group, and a sucrose + D-psicose administered group.

The two groups of rats were allowed free access to experiment feed (Table 2) and water, and maintained under specific conditions (temperature 22 ± 1°C, humidity 58 ± 2%, light period 8:00 to 20:00, dark period 20:00 to 8:00 next morning) . The energy metabolism was measured 5 to 7 weeks after the experiment feed was given. First, the rats from each group were individually put in a sealed portable desiccator (As One Corporation), and room air was continuously sent into the desiccator through a vent at 1.27 L/min, using an electromagnetic air pump (As One Corporation) . The rat breath and the room air were collected into a Douglas bag (Fukuda Sangyo) at 1-hour intervals, and the oxygen and carbon dioxide concentrations were measured with a gas concentration analyzers G-102 and LX-710 (Iijima Electronics Corporation) for 24 hours. The amounts of oxygen consumed by the rat, and the amount of generated carbon dioxide were calculated from the oxygen concentration and the carbon dioxide concentration in the breath, and the oxygen concentration and the carbon dioxide concentration in room air. Hourly energy expenditure was then calculated from the Weststrate equation.

The calculation result was divided by the measured rat weight. The results were represented as mean values and standard deviation, and a Student's t test was performed for significance testing. The results were considered significant for p < 0.05 (two sided).

**[Table 2]**

| Group | Sucrose g/kg | Sucrose + D-psicose g/kg |
|---|---|---|
| Sucrose | 649.9 | 649.9 |
| D-Psicose | 0.0 | 50.0 |
| Cellulose | 50.0 | 0.0 |
| Casein | 200.0 | 200.0 |
| DL-methionine | 3.0 | 3.0 |
| Soybean oil | 50.0 | 50.0 |
| Mineral mix (AIN-76) | 35.0 | 35.0 |
| Vitamin mix (AIN-76) | 10.0 | 10.0 |
| Choline chloride | 2.0 | 2.0 |
| Butylhydroxytoluene | 0.1 | 0.1 |
| | 1000 | 1000 |

### Results

FIG. 2 shows the energy expenditure data of rats in each time zone. The data are represented as energy expenditure of the rat per measured rat weight after 5 to 7 weeks to exclude the effect of rat size. The energy expenditure was significantly higher in the sucrose + D-psicose administered group than in the sucrose administered group at hour 11 in the light period, and at hour 5 and 6 in the dark period.

### [Example 2]

### Metabolism Test by Single Administration of D-Psicose in Humans

### Experiment method

Effects of single administration of D-psicose on energy expenditure, carbohydrate combustion, and lipid combustion were examined in humans. Energy metabolism was calculated by indirect calorimetry from measurements of subjects' breath (oxygen consumption and carbon dioxide discharge). The test was conducted by a single-blinded crossover study of two groups of 6 healthy individuals with no smoking habit (average age of 34.8; 3 males and 3 females; average BMI 21.4 kg/m²). Prior to the experiment, the subjects were fully informed of the experiment contents in line with the guidelines of the Declaration of Helsinki, and the experiment was conducted with the informed consent of all subjects.

The subjects were fasted for 12 hours before starting the test, and were each given a test solution (150 ml) prepared by adding 5 g of D-psicose, or a test solution (150 ml) prepared by adding 10 mg of high sweetener aspartame to provide the same sweetness as control food. The concentration of the test solution was 3.3% for the D-psicose group, and 0.005% for the aspartame group. After giving the test solution, the subjects were given breakfast (hamburg steak with rice; total calorie: 571 kcal; protein 13.4%, lipid 25.3%, carbohydrate 61.3%).

After the breakfast, the subjects were rested on the bed for 20 min in the resting supine position to acclimate to the environment, and the breath was measured with an Aero Monitor AE-310S (Minato Medical Science) connected to a breathing hood. The measurement was ended after 4 hours from the breakfast. During the measurement, the oxygen consumption and the carbon dioxide discharge of each subject were recorded every minute, and energy expenditure, carbohydrate combustion, and lipid combustion were calculated from the measured values using the Elwyn energy equation. Throughout the breath measurement, the subjects were rested on the bed in the resting supine position, and were kept awake and fasted.

The results were divided by subject's body weights, and were represented as mean values and standard deviation. A paired Student's t test was performed for significance testing. The results were considered significant for p < 0.05 (two sided).

### Results

FIG. 3 shows the energy expenditure, carbohydrate combustion, and lipid combustion per body weight of the subject during a time period after 20 minutes to 4 hours from breakfast, representing the effects of D-psicose on each energy metabolism parameter in humans. Compared to the control, the energy expenditure and the lipid combustion significantly increased when D-psicose was given immediately before eating the common lipid-containing food. The D-Psicose intake did not show any difference in the result when the dose was 2.4 g.

### [Example 3]

### Sensory Test by Continuous Administration of D-Psicose in Humans

### Experiment Methods

The test was conducted for 5 healthy individuals with no smoking habit (average age of 35.0; 3 males and 2 females) . Prior to the experiment, the subjects were fully informed of the experiment contents in line with the guidelines of the Declaration of Helsinki, and the experiment was conducted with the informed consent of all subjects.

The subjects were each asked to continuously take sample A (D-psicose, 1.0 g), once a day for 2 weeks. The subjects were then interviewed to see if there was any physical changes. After 1 week, the same subjects were continuously given sample B (D-psicose, 2.5 g) for 2 weeks in the same manner as for sample A, and were interviewed again. The same procedure was repeated after 1 week by continuously giving sample C (D-psicose, 5.0 g) to the same subjects, followed by an interview.

### Results

The interview results are shown in the table 3. The subjects all experienced some changes and felt that the body was "lighter","warmer", and "easier to move" after the continuous intake of 2.5 g or more of D-psicose. Some subjects felt "less fatigue", and had a "better feeling".

An experiment conducted by continuously giving sample A for 2 weeks after 1 week from testing with sample C produced the same result.

### Conclusion

The continuous administration of 2.5 g or more of D-psicose for 2 weeks was shown to increase or improve body temperature and other sensations in humans.

**[Table 3]**

| Sample | No change | Changes | | | | |
|---|---|---|---|---|---|---|
| | | Body felt lighter | Body felt warmer | Body became easier to move | Felt better | Felt less fatigue |
| A | 4 | 1 | 1 | 1 | 1 | 1 |
| B | 0 | 3 | 3 | 2 | 2 | 2 |
| C | 0 | 5 | 5 | 3 | 2 | 2 |

### Industrial Applicability

The invention should provide use of D-psicose for providing at least one selected from the group including an agent for preventing and/or improving reduced basal energy expenditure, an agent for preventing and/or improving reduced basal body temperature.

## Claims

1. An agent comprising D-psicose as an active ingredient for use in prevention and treatment of diseases involving low basal metabolism and low body temperature by accelerating the energy expenditure and/or improving the diminished energy expenditure functionality.

2. The agent for the use according to claim 1, wherein the agent is added to a food and drink product, a feed, a drug, a quasi drug, an oral composition, a cosmetic, or a food and drink additive so that the D-psicose is taken in the form of a food and drink product, a feed, a drug, a quasi drug, an oral composition, a cosmetic, or a food and drink additive in 2.5 g or more per administration per unit preparation for adults.

3. The agent for the use according to claim 1 or 2, wherein the agent is given to continuously administer the D-psicose at least once a day in 2.5 g or more for adults.

4. The agent for the use according to any one of claims 1 to 3, wherein the D-psicose contained as an active ingredient results from alkali isomerization of glucose, fructose, and/or an isomerized sugar.

5. The agent for the use according to any one of claims 1 to 4, wherein the D-psicose comprises D-psicose and/or derivatives thereof, the derivatives of D-psicose being selected from sugar alcohols in which the ketone group of D-psicose is converted into an alcohol group, uronic acids in which the alcohol group of D-psicose is oxidized, and amino sugars in which the alcohol group of D-psicose is substituted with an NH₂ group.

6. The agent for the use according to any one of claims 1 to 5, wherein the agent is given to administer the D-psicose for at least 2 weeks with food in a daily dose of 2.5 g to 25 g for adults.

7. The agent for the use according to any one of claims 1 to 6, wherein the agent is given to humans and other mammals in need of treatment and/or prevention of accelerated energy expenditure and/or diminished energy expenditure functionality.

## Patentansprüche

1. Mittel, das D-Picose als Wirkstoff enthält, für die Verwendung bei der Vorbeugung und Behandlung von Krankheiten, die mit einem niedrigen Grundumsatz und einer niedrigen Körpertemperatur verbunden sind, durch Beschleunigung des Energieverbrauchs und/oder Verbesserung der verminderten Funktionalität des Energieverbrauchs.

2. Mittel für die Verwendung nach Anspruch 1, wobei das Mittel einem Lebensmittel- und Getränkeprodukt, einem Futtermittel, einem Arzneimittel, einem Quasi-Arzneimittel, einer oralen Zusammensetzung, einem Kosmetikum oder einem Lebensmittel- und Getränkezusatzstoff zugesetzt ist, so dass die D-Picose in Form eines Lebensmittel- und Getränkeprodukts, eines Futtermittels, eines Arzneimittels, eines Quasi-Arzneimittels, einer oralen Zusammensetzung, eines Kosmetikums oder eines Lebensmittel- und Getränkezusatzstoffs für Erwachsene in einer Menge von 2,5 g oder mehr pro Verabreichung pro Zubereitungseinheit eingenommen wird.

3. Mittel für die Verwendung nach Anspruch 1 oder 2, wobei für Erwachsene das Mittel zur kontinuierlichen Verabreichung der D-Picose mindestens einmal täglich zu 2,5 g oder mehr gegeben wird.

4. Mittel für die Verwendung nach einem der Ansprüche 1 bis 3, wobei die als Wirkstoff enthaltene D-Picose durch Alkaliisomerisierung von Glucose, Fructose und/oder einem isomerisierten Zucker entsteht.

5. Mittel für die Verwendung nach einem der Ansprüche 1 bis 4, wobei die D-Psicose D-Psicose und/oder Derivate davon umfasst, wobei die Derivate der D-Psicose ausgewählt sind aus Zuckeralkoholen, bei denen die Ketongruppe der D-Psicose in eine Alkoholgruppe umgewandelt ist, Uronsäuren, bei denen die Alkoholgruppe der D-Psicose oxidiert ist, und Aminozuckern, bei denen die Alkoholgruppe der D-Psicose mit einer NH₂-Gruppe substituiert ist.

6. Mittel für die Verwendung nach einem der Ansprüche 1 bis 5, wobei für Erwachsene das Mittel zur Verabreichung der D-Psicose für mindestens 2 Wochen mit der Nahrung in einer täglichen Dosis von 2,5 g bis 25 g verabreicht wird.

7. Mittel für die Verwendung nach einem der Ansprüche 1 bis 6, wobei das Mittel Menschen und anderen Säugetieren zur Behandlung und/oder Vorbeugung von beschleunigtem Energieverbrauch und/oder verminderter Energieverbrauchsfunktionalität verabreicht wird.

## Revendications

1. Agent comprenant du D-psicose en tant que principe actif destiné à être utilisé dans la prévention et le traitement de maladies impliquant un métabolisme basal bas et une température corporelle basse par accélération de la dépense énergétique et/ou amélioration de la fonctionnalité de dépense énergétique diminuée.

2. Agent destiné à être utilisé selon la revendication 1, dans lequel l'agent est ajouté à un aliment et une boisson, un aliment pour animaux, un médicament, un quasi-médicament, une composition à usage oral, un cosmétique, ou un additif pour aliment et boisson, de façon que le D-psicose soit pris sous la forme d'un aliment et d'une boisson, d'un aliment pour animaux, d'un médicament, d'un quasi-médicament, d'une composition à usage oral, d'un cosmétique, ou d'un additif pour aliment et boisson à raison de 2,5 g ou plus par administration par préparation unitaire pour des adultes.

3. Agent destiné à être utilisé selon la revendication 1 ou 2, dans lequel l'agent est donné pour une administration continue du D-psicose au moins une fois par jour à raison de 2,5 g ou plus pour des adultes.

4. Agent destiné à être utilisé selon l'une quelconque des revendications 1 à 3, dans lequel le D-psicose contenu en tant que principe actif résulte de l'isomérisation alcaline du glucose, du fructose, et/ou d'un sucre isomérisé.

5. Agent destiné à être utilisé selon l'une quelconque des revendications 1 à 4, dans lequel le D-psicose comprend du D-psicose et/ou des dérivés de celui-ci, les dérivés de D-psicose étant choisis parmi les alcools de sucre dans lesquels le groupe cétone du D-psicose est converti en un groupe alcool, les acides uroniques dans lesquels le groupe alcool du D-psicose est oxydé, et les amino-sucres dans lesquels le groupe alcool du D-psicose est substitué par un groupe NH₂.

6. Agent destiné à être utilisé selon l'une quelconque des revendications 1 à 5, dans lequel l'agent est donné pour que le D-psicose soit administré pendant au moins 2 semaines avec un aliment à une dose quotidienne de 2,5 g à 25 g pour des adultes.

7. Agent destiné à être utilisé selon l'une quelconque des revendications 1 à 6, dans lequel l'agent est donné à des humains et d'autres mammifères ayant besoin d'un traitement et/ou d'une prévention d'une dépense énergétique accélérée et/ou d'une fonctionnalité de dépense énergétique diminuée.
